# EUROPÄISCHE PATENTSCHRIFT

(11) **EP 2 991 700 B1**
(45) Veröffentlichungstag und Bekanntmachung des Hinweises auf die Patenterteilung: **05.12.2018**
(21) Anmeldenummer: 14728464.0
(22) Anmeldetag: 05.05.2014
(51) Int. Cl.: A61M 3/02, A61H 23/02, A61H 35/00

(54) **FREQUENZGESTEUERTER SPÜLAPPARAT FÜR KÖRPERÖFFNUNGEN, INSBESONDERE DEN GEHÖRGANG**
FREQUENCY-CONTROLLED WASHING APPARATUS FOR BODILY ORIFICES, IN PARTICULAR THE AUDITORY CANAL
APPAREIL DE NETTOYAGE À COMMANDE FRÉQUENTIELLE POUR DES ORIFICES CORPORELS, NOTAMMENT POUR LE CONDUIT AUDITIF

(30) Priorität: 03.05.2013 DE 102013104535
(43) Veröffentlichungstag der Anmeldung: 09.03.2016
(73) Patentinhaber: Grimm, Peter, CH-3098 Schlieren/Könitz (CH)
(72) Erfinder: Grimm, Peter, CH-3098 Schlieren/Könitz (CH)
(74) Vertreter: Patentanwälte und Rechtsanwalt Weiß, Arat & Partner mbB
(86) Internationale Anmeldenummer: PCT/EP2014/059038
(87) Internationale Veröffentlichungsnummer: WO 2014/177712

(56) Entgegenhaltungen:
- EP-A1- 2 412 394
- DE-A1-102005 037 058
- US-B1- 7 699 820

## Beschreibung

### Technisches Gebiet

Die Erfindung betrifft eine Vorrichtung nach dem Oberbegriff des Anspruchs 1.

### Stand der Technik

Das Reinigen eines Ohrs kann in die Reinigung der Ohrmuschel und die Reinigung des Gehörgangs gegliedert werden. Die Ohrmuschel kann z.B. durch Wattestäbchen, feuchte Wattepads oder feuchte Tücher, wie z.B. Waschlappen, gereinigt werden.

In diesem Zusammenhang ist auf folgende Druckschriften zu verweisen, die Vorrichtungen und Verfahren zur Reinigung der Ohrmuschel und des Gehörgangs offenbaren: DE 10 2005 037 058 A1; EP 2 412 394 A1; US 7,699,820 B1.

Bei den bisherigen angewandten Reinigungen und Spülvorgängen des Ohrs durch die Druckpumpe oder Wattestäbchen kommt es bei Patienten häufig bereits nach kurzer Zeit zu erneuten Problemen mit Ablagerungen im Gehörgang, die durch eine erhöhte Produktion von Ohrenschmalz hervorgerufen werden. D.h. die Patienten können durch diese Arten der Ohrreinigung nicht dauerhaft von ihren Beschwerden befreit werden.

Zudem sind Wattestäbchen für Selbstanwendungen und zur Reinigung des Gehörgangs ungeeignet, da es durch das Nutzen dieser zu Verletzungen innerhalb des Gehörgangs, insbesondere des Trommelfells, kommen kann. Des Weiteren kann die Nutzung von Wattestäbchen zu einer Pfropfenbildung vor dem Trommelfell führen und/oder Entzündungen im Gehörgang verursachen. Dasselbe gilt für die Nutzung von Duschbrausen, Büroklammern, Zündhölzern oder ähnlichen Gegenständen, die als Hilfsmittel zur Reinigung des Gehörgangs zweckentfremdet werden. Auch die Selbstanwendung von Ohrensprays kann gefährliche Auswirkungen haben. So kann es durch die Nutzung dieser Sprays bei Vorerkrankungen des Ohrs zu sehr starken Entzündungen des Ohrs oder starkem Schwindelgefühl kommen.

Der Gehörgang kann z.B. mittels Ohr-Wasser-Reinigungsgeräten gereinigt werden. Diese Methode wird vor allem bei akuten Krankheitserscheinungen, wie dem Hörsturz, angewendet. Dabei handelt es sich jedoch weniger um einen aktiven Reinigungsakt, sondern um ein Durchströmen des Aussenohrs mit warmem Wasser, was zu einer Durchblutungsförderung im Gehörgang führen soll.

Durch Ohrenkerzen soll durch Selbstanwendung ebenfalls die Durchblutung im Gehörgang gefördert werden, wodurch die natürliche Selbstreinigung des Ohres verbessert werden soll. Weiterhin soll durch einen entstehenden Unterdruck im Gehörgang, dieser von überflüssigem Schmalz befreit werden. Dies ist durch den zu geringen Unterdruck jedoch nicht der Fall. Weiterhin kann es bei der Nutzung zu Verletzungen des Ohrs, insbesondere des Trommelfells kommen, die durch Verbrennungsrückstände der Kerzen hervorgerufen werden.

Zur Reinigung des Gehörgangs sind sowohl Vorrichtungen die das ganze Ohr umschliessen und mittels einer Gummidichtung nach aussen abdichten, als auch Vorrichtungen die auf den Gehörgang aufgesetzt werden und diesen über einen Silikonaufsatz nach aussen abdichten, bekannt. Bei diesen Vorrichtungen wird der Gehörgang zur Reinigung ganz mit Wasser und Reinigungsmittel gefüllt.

Bei Vorrichtungen, die das ganze Ohr umschliessen wird eine mit Wasser und Reinigungsmittel gefüllte Schale an das Ohr gepresst und dadurch der Gehörgang mit der Flüssigkeit gefüllt. Zur Verbesserung der Reinigungsleistung wird das Wasser-/Reinigungsgemisch über eine elektrisch betriebene Membran in Schwingung versetzt. Diese Schwingungen und die Wasserbewegungen können jedoch einen Druck auf das Trommelfell aufbauen, welcher schädigende Wirkungen haben kann. Aus diesem Grund müssen bei diesen Vorrichtungen der Druck und dadurch die Schwingungen klein gehalten werden, wodurch die Reinigungsleistung reduziert und somit uneffizient wird.

Bei Vorrichtungen, die auf den Gehörgang aufgesetzt werden, wird die mit Wasser-/Reinigungsgemisch gefüllte Vorrichtung direkt auf den Gehörgang aufgesetzt und dieser dadurch mit der Flüssigkeit gefüllt. Bei diesen Vorrichtungen wird zur Verbesserung der Reinigungsleistung das Wasser-/Reinigungsgemisch mittels eines elektrischen Motors und an einer Antriebswelle angebrachter Paddel in Rotation versetzt. In anderen Ausführungsbeispielen, die direkt am Gehörgang aufgesetzt werden, wird die Reinigungsflüssigkeit über eine Druck-Saug-Vorrichtung in den Gehörgang gepresst und wieder abgesaugt. Dabei kann die Vorrichtung z.B. als Spritze, Pipette oder Ballon ausgebildet sein. Durch das Einbringen der Reinigungsflüssigkeit in den Gehörgang kann es bei diesen Methoden vor allem zu Verletzungen des Trommelfells kommen.

### Aufgabe der Erfindung

Aufgabe der Erfindung ist es, eine Vorrichtung für regelmässige Selbstanwendungen und zur schonenden Pflege und Reinigung einer Körperöffnung eines Lebewesens, insbesondere des Gehörgangs des menschlichen Ohrs zur Verfügung zu stellen. Dabei soll durch nachhaltige Auflösung von Verschmutzungen im Gehörgang, zur Vermeidung von Verstopfungen, Entzündungen und Schäden verhindert bzw. vermieden werden. Dabei soll zur Erhaltung des natürlichen Schutzfilms des Ohrs eine Anwendung nur alle fünf bis zehn Tage stattfinden. Des Weiteren soll durch die patientenspezifische individuelle Anpassung einer bestimmten Impulsfrequenz des Apparats durch einen Arzt die Gefahr einer Schädigung durch zu hohen Druck im Gehörgang des Anwenders verhindert werden.

Weiterhin sollte die Erfindung kompakt, handlich, preislich günstig und in der Anwendung einfach zu verstehen sein, damit diese neben dem Einsatz im medizinischen Bereich durch Ärzte auch im häuslichen Gebrauch bei der Selbstanwendung zum Einsatz kommen kann.

### Lösung der Aufgabe

Zur Lösung der Aufgabe führen die Merkmale nach dem Anspruch 1.

In typischen Ausführungsbeispielen umfasst eine Vorrichtung zur Reinigung einer Körperöffnung, insbesondere des Gehörgangs eines Lebewesens ein flüssiges Medium. Bei dem flüssigen, Medium handelt es sich vorzugsweise um Wasser oder um ein Flüssigkeitsgemisch bestehend aus Wasser und einem Reinigungsmittel und/oder einer Pflegelotion. Um den Reinigungseffekt zu erhöhen und die Nutzung für den Anwender angenehmer zu gestalten, handelt es sich dabei vorzugsweise um ein warmes flüssiges Medium in einem Temperaturbereich von 30°C bis 40°C, besser in einem Temperaturbereich von 35°C bis 38°C.

Weiterhin ist die Nutzung eines Flüssigkeitsgemischs aus Wasser und einem abrasiven Mittel möglich. Durch die Nutzung eines abrasiven Mittels kann die Reinigungsleistung verbessert und die Durchblutung des Gehörgangs gefordert werden. Diese Vorteile entstehen dadurch, dass kleine Partikel des abrasiven Mittels durch Mikroturbulenzen gegen die Aussenwand des Gehörgangs und den Schmutzfilm im Gehörgang geschleudert werden. Zudem soll auch die Nutzung eines desinfizierenden Mittels zu einer prophylaktischen Behandlung denkbar und vorliegend umfasst sein.

In typischen Ausführungsbeispielen wird das flüssige Medium in einem zur proximalen Seite offenem Gehäuse aufgenommen. Dabei ist die Form des Gehäuses zur Aufnahme des Mediums unerheblich, vorzugsweise handelt es sich dabei zur besseren Handhabung um ein becher-, schalen- oder muschelartig geformtes Gehäuse. Dieses Gehäuse dient primär zur Aufnahme des Mediums zur Reinigung des Gehörgangs und kann daher prinzipiell aus den unterschiedlichsten Materialien bestehen, vorzugsweise aus einem Kunststoff oder Metall. Je nach Konstruktion kann die Schale zur Aufnahme des Reinigungsmediums auch dehnbar gestaltet sein. Des Weiteren weist der Werkstoff des Gehäuses eine Beständigkeit gegen heisses Wasser, Säuren und/oder Laugen auf und stellt keine Belastung für den Organismus dar. Daher ist vorzugsweise der Einsatz thermoplastischer Kunststoffe für das Gehäuse denkbar.

An der zum Körper hin offenen Seite schliesst das Gehäuse mit einer Dichtung ab, die vorzugsweise aus Gummi bestehen kann, jedoch sollen auch andere Dichtungsstoffe vorliegend umfasst sein, um eventuelle Unverträglichkeiten gegenüber Stoffen von Anwendern vorzubeugen. Durch diese Dichtung soll weiterhin ein Austreten des Reinigungsmediums im Anwendungsfall verhindert werden.

Im Boden des Gehäuses, d.h. auf der distalen Seite des Gehäuses. verschliesst eine austauschbare Abdeckung auf einer proximalen Seite des Gehäusebodens eine Öffnung im Gehäuseboden. Diese Abdeckung verhindert zudem, dass andere Teile der Vorrichtung, ausser der Gehäuseschale, mit dem in der Gehäuseschale befindlichen Medium in Kontakt kommen. Die Abdeckung sollte zudem aus einem elastischen Werkstoff bestehen. Auf diese Weise können die von dem Impulsnocken erzeugten Schwingungen auf das flüssige Medium in der Gehäuseschale übertragen werden. Der Werkstoff sollte, wie das Gehäuse, eine Beständigkeit gegen heisses Wasser, Säuren und/oder Laugen aufweisen.

Im Gegensatz zu Systemen mit einer Membran, bei denen der gesamte Gehäuseboden die Schwingungen erzeugt und somit sämtliches eingeschlossenes Medium in Schwingung versetzt wird, arbeitet die erfindungsgemässe Vorrichtung auf eine andere Art und Weise.

Bei der erfindungsgemässen Vorrichtung wird der Impuls über einen, im Wesentlichen mittig im Gehäuseboden befindlichen Impulsnocken erzeugt. Bei diesem Impulsnocken kann es sich um einen kleinen Bolzen handeln, der senkrecht zum Gehäuseboden betätigt wird. Es ist aber auch vorgesehen, dass es sich um ein waagrecht wirkendes Pendel handeln.

Messungen haben die Unbedenklichkeit der Druckverhältnisse gezeigt. Der wissenschaftliche mediane Repturdruck eines Trommelfells liegt bei 912 mmHg und dieses Anwendungssystem erzeugt in der Versuchsanordnung lediglich um ∼ 22 mmHg. Das bedeutet, dass der Druck für das Trommelfell bis vierzig Mal geringer ist als der mittlere Repturdruck mit Gefahr für das Trommelfell.

Die Druckschwingungen, mit denen der Impulsnocken in Schwingung versetzt wird, können sowohl mechanisch als auch elektronisch durch eine Schwingungsenergiequelle, die einer Antriebseinheit entspricht, hervorgerufen werden. Dabei ist die Schwingung mechanisch, z.B. über den Einsatz von Mikrokolben, Pleuel oder Unwucht und elektronisch durch die Nutzung des Lautsprecherprinzips oder eines Mikrochips erreichbar.

Weiterhin beinhaltet die Antriebseinheit eine programmierbare Steuerung über die die Frequenz und Amplitude der Schwingungen des Impulsnockens auf die Eigenschwingung des Gehörgangs, des jeweiligen Anwenders, durch medizinisches Fachpersonal eingestellt werden kann. Hierzu muss der Gehörgang, durch medizinisches Fachpersonal vermessen werden um darauf sowohl Frequenz als auch Amplitude, der durch die Vorrichtung erzeugten Interferenzschwingungen, auf den jeweiligen späteren Anwender anzupassen. Dadurch können Verletzungen, z.B. des Trommelfells, durch zu hohen Druck vermieden werden.

Zusätzlich zu der Frequenz für die Reinigung des Gehörgangs soll in einem typischen Ausführungsbeispiel der Vorrichtung die Einstellung einer höheren Frequenz, die zur Massage des Umgebungsgewebes genutzt werden kann, möglich sein. Diese ist ebenfalls über die Steuereinheit auf den jeweiligen Anwender durch Fachpersonal individuell einstellbar. Denkbar ist ebenfalls, dass in einer Vorrichtung mehrere Eigenfrequenzen von verschiedenen Gehörgängen, z.B. für den Einsatz in Familien, programmiert werden können.

Die Antriebseinheit wird in einer typischen Ausführung über einen Multischalter bedient. Dies hat den Vorteil, dass die Vorrichtung über den Schalter ein- und ausgeschaltet werden kann und zudem die Programmwahl über den Schalter erfolgen kann. Weiterhin ist dadurch auch die Möglichkeit der Auswahl verschiedener Eigenfrequenzen für verschiedene Nutzer denkbar. Jedoch soll auch die Möglichkeit zweier separater Schalter für Programmwahl und Aktivierung bzw. Deaktivierung der Vorrichtung vorliegend umfasst sein.

In typischen Ausführungsbeispielen ist für die Energieversorgung der Antriebseinheit vorzugsweise eine elektrische Batterie vorgesehen. Um den Apparat kompakt zu halten, handelt es sich vorzugsweise um Primärzellen, wie z.B. Lithium-Knopfzellen oder um Akkumulatoren, welche im Gegensatz zur Primärzelle den Vorteil der Möglichkeit des Wiederaufladens besitzen. Der Einsatz einer elektrischen Batterie gegenüber einer Direktversorgung über ein Netzteil ist, dass die Vorrichtung dadurch uneingeschränkter nutzbar ist und zudem durch den Entfall des Netzteils kompakter, handlicher und preislich günstiger ist.

Bei einer typischen Verwendung der Vorrichtung wird zunächst die Gehäuseschale mit einem flüssigen Medium befüllt. Danach wird die Vorrichtung an eine Körperöffnung, insbesondere dass Ohr angesetzt und so positioniert, dass sich die Körperöffnung mit dem sich in der Gehäuseschale befindlichen Medium füllt. Der nächste Schritt ist die Aktivierung der Vorrichtung über einen Multischalter sowie die anschliessende Programmwahl über den Multischalter.

In typischen Ausführungsbeispielen erfolgt die Reinigung und Pflege des Gehörgangs durch gesteuerte Impulswellen, die durch den Impulsnocken gezielt und konzentriert auf einen Punkt gerichtet werden. Das heisst, dass nicht das gesamte flüssige Medium in Schwingung gebracht wird. Vielmehr wird durch die Impulswellen innerhalb des flüssigen Mediums eine aktivierte Wassersäule erzeugt. Das dabei bewegte Volumen des flüssigen Mediums beträgt zwischen 100 mm³ und 300 mm³. Durch diese, auf die Eigenfrequenz des Gehörgangbaus abgestimmten Schwingungen, entstehen pulsierende Stosswellen. Die Stosswellen führen dazu, dass der Gehörgang nicht vollständig mit dem angewandten flüssigen Medium gefüllt ist, sondern das Luft-/Flüssigkeits-Mikroturbulenzen entstehen, durch die im Gehörgang eine Spül- und Lösewirkung hervorgerufen wird, deren Resultat die Reinigung des Gehörgangs darstellt.

In typischen Ausführungsbeispielen werden die Interferenzschwingungen durch das gezielte Wirken des Impulsnockens auf eine minimale Fläche erzeugt, Um die pulsierende Wassersäule gezielt auf den Gehörgang wirken zu lassen liegt die Grösse der Fläche in einem Bereich von 0,5 cm² bis 1,0 cm² und beträgt vorzugsweise 0,8 cm². Die Amplitude der Schwingungen kann über den Hub des Impulsnockens gesteuert werden und kann, durch medizinisches Fachpersonal auf den Gehörgang des Anwenders angepasst, in einem Bereich zwischen 0,25 mm und 1,0 mm eingestellt werden. Die Frequenz für die Schwingungen zur Pflege und Reinigung des Gehörgangs liegen im Bereich der Eigenfrequenz des Gehörgangbaus von 8 Hz bis 15 Hz. Für die Massage des Umgebungsgewebes kann die Frequenz der Schwingungen in einem Bereich zwischen 16 Hz und 300 Hz liegen.

Obwohl die erfindungsgemässe Vorrichtung für die Reinigung und Pflege eines menschlichen Gehörgangs beschrieben wurde, kann diese ebenso bei anderen Körperöffnungen und/oder anderen Lebewesen angewandt werden.

### Figurenbeschreibung

Weitere Vorteile, Merkmale und Einzelheiten ergeben sich aus der nachfolgenden Beschreibung bevorzugter Ausführungsbeispiele sowie anhand der Figuren. Diese zeigen in
- Figur 1: eine schematische Darstellung einer erfindungsgemässen Vorrichtung zur Reinigung einer Körperöffnung, insbesondere des Gehörgangs, sowohl von der proximalen als auch der distalen Seite.
- Figur 2: die schematische Darstellung der Vorrichtung in der Ausführung mit einem waagrecht wirkenden Pendel bzw. Schwingarm
- Figur 3: die schematische Darstellung der Vorrichtung in der Ausführung mit einem senkrecht betätigten Bolzen; und
- Figur 4: einen schematischen koronaren Schnitt durch die Vorrichtung und den Bereich des Ohrs, in dem die erfindungsgemässe Vorrichtung in Gebrauchslage im Anwendungsfall dargestellt wird.

### Ausführungsbeispiel

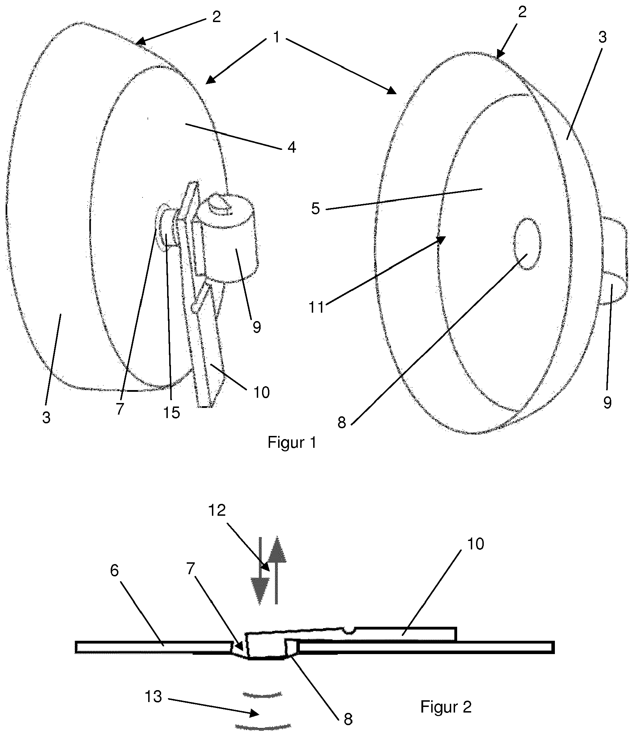

Figur 1 zeigt schematisch eine erfindungsgemässe Vorrichtung 1. Die Vorrichtung 1 umfasst ein Gehäuse 2. Auf einer distalen Seite des Gehäuses 2 befindet sich ein Gehäuseboden 6 mit einer Öffnung 7. Auf der proximalen Seite des Gehäusebodens 6 befindet sich eine austauschbare elastische Abdeckung 8. Auf einer distalen Seite der Abdeckung 8 befindet sich ein Impulsnocken 15 sowie eine Antriebseinheit 9.

Durch die Abdeckung 8 auf der proximalen Seite des Gehäusebodens 6 wird einr Hohlraum 11 zur Aufnahme eines flüssigen Mediums 16 von den auf der distalen Seite des Gehäuses 2 und der Abdeckung 8 befindlichen Komponenten abgedichtet.

Weiterhin umfasst die Vorrichtung 1 in den Figuren nicht näher dargestellte Elemente. Bei diesen handelt es sich um eine Steuerung, einen Multischalter, die Bauteile der Energieversorgung sowie weitere Bauteile zur Verbindung der einzelnen Komponenten. Als Antriebseinheit bzw. Schwingungserzeuger 9 des Impulsnockens 15 können mechanische Systeme, wie z.B. Mikrokolben, Pleuel und/oder Unwucht oder elektronische Systeme, wie z.B. ein Mikrochip und/oder eine Ansteuerung nach dem Lautsprecherprinzip zum Einsatz kommen.

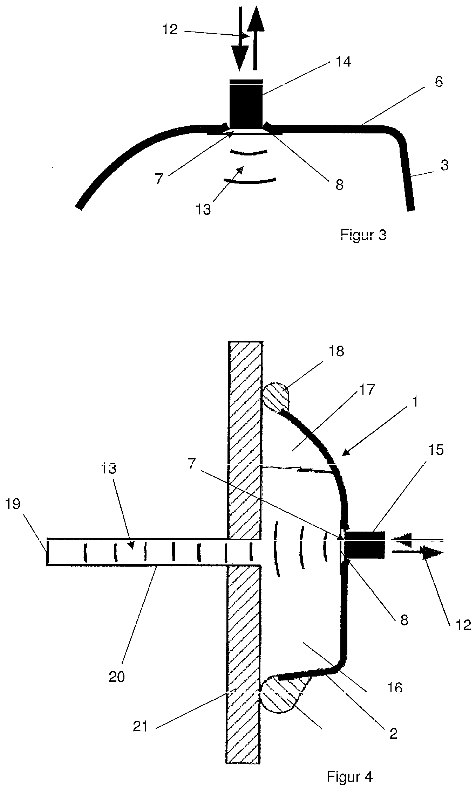

Der Impulsnocken 15 kann, dargestellt in Figur 2, waagrecht als Pendel 10 wirken oder, dargestellt in Figur 3, senkrecht als Bolzen ausgeformt sein. Die Frequenz und Amplitude mit denen der Impulsnocken 15 schwingt, kann über die Steuerung durch medizinisches Fachpersonal eingestellt werden. Die Energieversorgung erfolgt über nicht wiederaufladbare Primärzellen, z.B. Lithium-Knopfzellen oder über wiederaufladbare Akkumulatoren. Bei dem Schalter handelt es sich vorzugsweise um einen Multischalter zur Auswahl der einzelnen Funktionen. Die nicht dargestellten Bauteile sind bei einer erfindungsgemässen Vorrichtung 1 vorzugsweise am Gehäuse 2 angebracht. Auch eine Bauweise, in der Antriebseinheit 9, Batteriefach, Steuerung und Schalter innerhalb eines erweiterten Gehäuses 2 bzw. einer Abdeckung des Gehäuses 2 angebracht sind, soll vorliegend umfasst sein. Die Antriebseinheit 9 kann sowohl mechanisch, z.B. über Mikrokolben, Pleuel oder Unwucht, oder elektrisch. z.B. über das Lautsprecherprinzip oder einen Mikrochip, angetrieben werden.

Zur Aufnahme eines Mediums bilden eine Gehäusewand 3 und der Gehäuseboden 6 einen auf der proximalen Seite geöffneten Behälter, der insbesondere der Aufnahme des flüssigen Mediums zur Reinigung des Gehörgangs dient. Dieser schliesst auf der proximalen Seite vorzugsweise mit einer Dichtung 18 ab. Die Dichtung 18 kann aus verschiedenen Stoffen, wie z.B. Gummi bestehen. Bei der Ausführung der Form des Gehäuses 2 durch einen dehnfähigen Kunststoff ist diese Dichtung 18 nicht zwingend erforderlich, da hier eine ausreichende Dichtwirkung durch die mögliche Formänderung des Kunststoffs angenommen werden kann.

Die Form und Grösse des Gehäuses 2 ist im Grunde unerheblich. In einer erfindungsgemässen Ausführung der Vorrichtung ist jedoch die Wahl einer becher- oder schalenartigen Form, wie in Figur 1 dargestellt, oder einer muschelartigen Form, wie in Figur 4 dargestellt, auf Grund der besseren Handhabung durch den Anwender anderen Ausführungsformen vorzuziehen.

Die Öffnung 7 im Gehäuseboden 6 ist, wie der Impulsnocken 15 in der jeweiligen Grösse anpassbar, sodass nur eine definierte Wassersäule durch die gerichteten Impulswellen 13 zur Erzeugung der Luft-/Flüssigkeits-Mikroturbulenzen in Schwingung versetzt wird. Diese Interferenzschwingungen sind auf einen Gehörgang 20 des Anwenders angepasst um eine Schädigung von diesem und/oder einem Trommelfell 19 zu vermeiden.

Figur 4 stellt schematisch die erfindungsgemässe Vorrichtung 1 im Anwendungsfall an einem menschlichen Ohr dar. Um in diese Position zu gelangen, wird das Gehäuse 2 mit einem flüssigen Medium 16 befüllt. Bei diesem handelt es sich vorzugsweise um warmes Wasser oder ein Gemisch aus warmen Wasser und einem Reinigungsmittel und/oder einem Pflegemittel.

Anschliessend wird die Vorrichtung 1 mit der proximalen Seite an einen geneigten Kopf 21 so angelegt, dass diese das Ohr umschliesst und der Hohlraum 11 im Gehäuse 2 mit dem Kopf 21 einen geschlossenen abgedichteten Raum bildet. Anschliessend wird der geneigte Kopf 21 aufgerichtet, wodurch das flüssige Medium 16 in den Gehörgang 20 gelangen kann.

Sobald sich die Vorrichtung 1 in der für die Anwendung vorgesehenen Position befindet, wird diese über den nicht näher dargestellten Multischalter in Betrieb gesetzt und zudem über den Multischalter eine Programmwahl getroffen Dadurch wird der Impulsnocken 15 durch die Antriebseinheit 9 in die zuvor angepasste Eigenfrequenz des Gehörgangs 20 oder die Frequenz für die Massage des Umgebungsgewebes gesetzt. Durch die pulsierenden gerichteten Impulswellen 13 entstehen daraufhin in dem flüssigen Medium 16 und durch eine in dem Hohlraum 11 zusätzlich eingeschlossene Luft 17 entsprechende Luft-/Flüssigkeits- Mikroturbulenzen. Durch diese Luft-/Flüssigkeits-Mikroturbulenzen wird im Gehörgang 20, eine für das Trommelfell 19 schonende Spül- und Lösewirkung hervorgerufen, durch welche der Gehörgang 20 von Schmutz und hartnäckigen Ablagerungen befreit wird.

**Bezugszeichenliste**

| | | | |
|---|---|---|---|
| 1 | Vorrichtung | 34 | |
| 2 | Gehäuse | 35 | |
| 3 | Gehäusewand | 36 | |
| 4 | Gehäuseboden (distale Seite) | 37 | |
| 5 | Gehäuseboden (proximale Seite) | 38 | |
| 6 | Gehäuseboden | 39 | |
| 7 | Öffnung im Gehäuseboden | 40 | |
| 8 | austauschbare, elastische Abdeckung | 41 | |
| 9 | Antriebseinheit | 42 | |
| 10 | Pendel/Schwingarm | 43 | |
| 11 | Hohlraum | 44 | |
| 12 | Bewegungen des Impulsnockens | 45 | |
| 13 | gerichtete Impulswellen | 46 | |
| 14 | Bolzen | 47 | |
| 15 | Impulsnocken (allgemein) | 48 | |
| 16 | Flüssiges Medium | 49 | |
| 17 | Luft | 50 | |
| 18 | Dichtung | 51 | |
| 19 | Trommelfell | 52 | |
| 20 | Gehörgang | 53 | |
| 21 | Kopf | 54 | |
| 22 | | 55 | |
| 23 | | 56 | |
| 24 | | 57 | |
| 25 | | 58 | |
| 26 | | 59 | |
| 27 | | 60 | |
| 28 | | 61 | |
| 29 | | 62 | |
| 30 | | 63 | |
| 31 | | 64 | |
| 32 | | 65 | |
| 33 | | 66 | |

## Patentansprüche

1. Vorrichtung (1) zur Reinigung einer Körperöffnung, insbesondere eines Gehörgangs (20), die ein auf einer proximalen Seite offenes Gehäuse (2) zur Aufnahme eines flüssigen Mediums (16) umfasst, wobei ein Gehäuseboden (6) auf einer distalen Seite des Gehäuses (2) einen Impulsnocken (15) umfasst,
**dadurch gekennzeichnet,**
**dass** sich auf der distalen Seite des Gehäuses (4) eine Öffnung (7) zur Aufnahme des Impulsnockens (15) befindet und dass sich auf einer proximalen Seite der Öffnung (7) eine elastische, austauschbare Abdeckung (8) befindet.

2. Vorrichtung nach Anspruch 1, **dadurch gekennzeichnet, dass** sich auf einer distalen Seite der Abdeckung (8) der Impulsnocken (15) befindet.

3. Vorrichtung nach Anspruch 1 und 2, **dadurch gekennzeichnet, dass** die Abdeckung (8) die proximale Seite des Gehäuses (5) von der distalen Seite des Gehäuses (4) abdichtet.

4. Vorrichtung nach einem der vorherigen Ansprüche, **gekennzeichnet dadurch, dass** die Vorrichtung eine Antriebseinheit aufweist, wobei Frequenz und Amplitude des Impulsnockens (15) über eine programmierbare Steuerung der Antriebseinheit (9) einstellbar sind.

5. Vorrichtung nach einem der vorherigen Ansprüche, **gekennzeichnet dadurch, dass** der Impulsnocken (15) zu dem Gehäuseboden (6) senkrecht als ein Bolzen (14) oder waagrecht als ein Schwingarm (10) angeordnet ist.

6. Vorrichtung nach Anspruch 4, **gekennzeichnet dadurch, dass** die Antriebseinheit (9) mechanisch oder elektronisch antreibbar ist.

7. Vorrichtung nach einem der vorherigen Ansprüche, **gekennzeichnet dadurch, dass** die Gehäuseschale (2) auf der proximalen Seite eine Dichtung (18) aufweist.

8. Vorrichtung nach einem der vorherigen Ansprüche, **gekennzeichnet dadurch, dass** ein Multischalter vorhanden ist.

## Claims

1. Device (1) for cleaning a bodily orifice, in particular an auditory canal (20), which device comprises a housing (2) that is open on a proximal side and that is intended to hold a liquid medium (16), wherein a housing bottom (6) comprises an impulse tappet (15) on a distal side of the housing (2),
**characterized**
**in that** an opening (7) for receiving the impulse tappet (15) is located on the distal side of the housing (4), and in that an elastic, replaceable cover (8) is located on a proximal side of the opening (7).

2. Device according to claim 1, **characterized in that** the impulse tappet (15) is located on a distal side of the cover (8) .

3. Device according to claim 1 or 2, **characterized in that** the cover (8) seals off the proximal side of the housing (5) from the distal side of the housing (4).

4. Device according to one of the preceding claims, **characterized in that** the device has a drive unit, wherein the frequency and amplitude of the impulse tappet (15) can be set via a programmable controller of the drive unit (9).

5. Device according to one of the preceding claims, **characterized in that** the impulse tappet (15), in relation to the housing bottom (6), is arranged vertically as a bolt (14) or horizontally as a swing arm (10).

6. Device according to claim 4, **characterized in that** the drive unit (9) can be driven mechanically or electronically.

7. Device according to one of the preceding claims, **characterized in that** the housing shell (2) has a seal (18) on the proximal side.

8. Device according to one of the preceding claims, **characterized in that** a multi-switch is present.

## Revendications

1. Dispositif (1) de nettoyage pour un orifice corporel, en particulier pour un conduit auditif (20), qui comporte un boîtier (2) ouvert d'un côté proximal pour recevoir un milieu liquide (16), dans lequel un fond de boîtier (6) comporte une came à impulsions (15) d'un côté distal du boîtier (2),
**caractérisé par le fait**
**que** du côté distal du boîtier (4) se trouve une ouverture (7) destinée à recevoir la came à impulsions (15) et que d'un côté proximal de l'ouverture (7) se trouve un couvercle résilient (8) interchangeable.

2. Dispositif selon la revendication 1, **caractérisé par le fait que** d'un côté distal du couvercle (8) se trouve la came à impulsions (15).

3. Dispositif selon les revendications 1 ou 2, **caractérisé par le fait que** le couvercle (8) sépare de manière étanche le côté proximal du boîtier (5) du côté distal du boîtier (4).

4. Dispositif selon l'une des revendications précédentes, **caractérisé par le fait que** le dispositif présente une unité d'entraînement, la fréquence et l'amplitude de la came à impulsions (15) étant réglables par l'intermédiaire d'une commande programmable de l'unité d'entraînement (9).

5. Dispositif selon l'une des revendications précédentes, **caractérisé par le fait que** la came à impulsions (15) est disposée verticalement au fond du boîtier (6) comme un boulon (14) ou horizontalement comme un bras pivotant (10).

6. Dispositif selon la revendication 4, **caractérisé par le fait que** l'unité d'entraînement (9) peut être entraînée mécaniquement ou électroniquement.

7. Dispositif selon l'une des revendications précédentes, **caractérisé par le fait que** la coque de boîtier (2) présente, du côté proximal, un joint (18).

8. Dispositif selon l'une des revendications précédentes, **caractérisé par le fait qu'**il est présent un multi-commutateur.
